# EUROPÄISCHE PATENTSCHRIFT

(11) **EP 1 524 905 B1**
(45) Veröffentlichungstag und Bekanntmachung des Hinweises auf die Patenterteilung: **26.05.2010**
(21) Anmeldenummer: 03764967.0
(22) Anmeldetag: 10.07.2003
(51) Int. Cl.: A01N 43/653, C07D 249/12

(54) **KRISTALLMODIFIKATION II DES 2- 2-(1-CHLOR-CYCLOPROPYL)-3-(2-CHLORPHENYL)-2-HYDROXY-PROPYL -2,4-DIHYDRO-3H-1,2,4-TRIAZOL-3-THIONS**
CRYSTAL MODIFICATION II OF 2- 2-(1-CHLORO-CYCLOPROPYL)-3-(2-CHLOROPHENYL)-2-HYDROXY-PROPYL -2,4-DIHYDRO-3H-1,2,4-TRIAZOLE-3-THIONS
MODIFICATION CRISTALLINE II DU 2- 2-(1-CHLOROCYCLOPROPYL)-3-(2-CHLOROPHENYL)-2-HYDROXYPROPYL -2,4-DIHYDRO-3H-1,2,4-TRIAZOL-3-THION

(30) Priorität: 22.07.2002 DE 10233171
(43) Veröffentlichungstag der Anmeldung: 27.04.2005
(73) Patentinhaber: Bayer CropScience AG, 40789 Monheim (DE)
(72) Erfinder: SEIDEL, Erika, 53639 Königswinter (DE); VERMEER, Ronald, 51371 Leverkusen (DE); HASENACK, Karin, 58332 Schwelm (DE); OLENIK, Britta, 46242 Bottrop (DE)
(86) Internationale Anmeldenummer: PCT/EP2003/007473
(87) Internationale Veröffentlichungsnummer: WO 2004/008860

(56) Entgegenhaltungen:
- WO-A-96/16048

## Beschreibung

Die vorliegende Erfindung betrifft die Kristallmodifikation II des 2-[2-(1-Chlor-cyclopropyl)-3-(2-chlorphenyl)-2-hydroxy-propyl]-2,4-dihydro-3H-1,2,4-triazol-3-thions, ein Verfahren zur Herstellung dieser Substanz sowie deren Verwendung zur Bekämpfung von unerwünschten Mikroorganismen.

Das 2-[2-(1-Chlor-cyclopropyl)-3-(2-chlorphenyl)-2-hydroxy-propyl]-2,4-dihydro-3H-1,2,4-triazol-3-thion und dessen Einsatz als Mikrobizid, insbesondere als Fungizid, ist bereits bekannt (vgl. WO 96-16 048). Ebenso ist bekannt, dass sich diese Substanz herstellen lässt, indem man 2-(1-Chlor-cyclopropyl)-1-(2-chlorphenyl)-3-(1,2,4-triazol-1-yl)-propan-2-ol entweder (a) mit Schwefel in Gegenwart von N-Methyl-pyrrolidon bei Temperaturen von etwa 200°C behandelt oder (b) zunächst mit n-Butyl-lithium in Gegenwart von Hexan und dann mit Schwefel in Gegenwart von Tetrahydrofuran umsetzt (vgl. WO 96-16 048). Wie sich jetzt gezeigt hat, kann der Wirkstoff in zwei verschiedenen Kristallmodifikationen anfallen, von denen die Modifikation I bei Raumtemperatur metastabil und die Modifikation II bei Raumtemperatur thermodynamisch stabil ist.

Das Auftreten von Wirkstoffen in verschiedenen Kristallformen (=Polymorphie) ist sowohl für die Ausarbeitung von Herstellungsverfahren als auch für die Entwicklung von Formulierungen von großer Bedeutung. So unterscheiden sich die verschiedenen Modifikationen einer chemischen Verbindung neben dem Aussehen (Kristallhabitus) und der Härte auch in zahlreichen weiteren physiko-chemischen Eigenschaften. Dabei können Unterschiede bezüglich der Stabilität, der Löslichkeit, der Hygroskopizität, des Schmelzpunktes, der Feststoffdichte und der Fließfähigkeit einen starken Einfluss auf die Qualität und die Wirksamkeit von Pflanzenbehandlungsmitteln ausüben. Es ist bisher nicht möglich, das Auftreten und die Anzahl von Kristallmodifikationen einschließlich ihrer physiko-chemischen Eigenschaften vorherzusagen. Vor allem die thermodynamische Stabilität und auch das unterschiedliche Verhalten nach Darreichung in lebenden Organismen lassen sich nicht im Voraus bestimmen.

Es ist allgemein bekannt, dass die verschiedenen Modifikationen einer Substanz monotrop oder enantiotrop vorliegen können. Im Fall der monotropen Polymorphie kann eine Kristallform über den gesamten Temperaturbereich bis zum Schmelzpunkt die thermodynamisch stabile Phase darstellen, wohingegen bei enantiotropen Systemen ein Umwandlungspunkt existiert, bei dem sich das Stabilitätsverhältnis umkehrt. Es ist nicht möglich, das Stabilitätsverhältnis insbesondere die Existenz und die Lage eines solchen Umwandlungspunktes vorherzusagen. Ein aktueller Überblick über den Stand des Wissens zu diesen prinzipiellen thermodynamischen Verhältnissen ist in Angew. Chem. Int. Ed. 1999, 38, 3440-3461 gegeben.

Es wurde nun die Kristallmodifikation II des 2-[2-(1-Chlor-cyclopropyl)-3-(2-chlorphenyl)-2-hydroxy-propyl]-2,4-dihydro-3H-1,2,4-triazol-3-thions der Formel 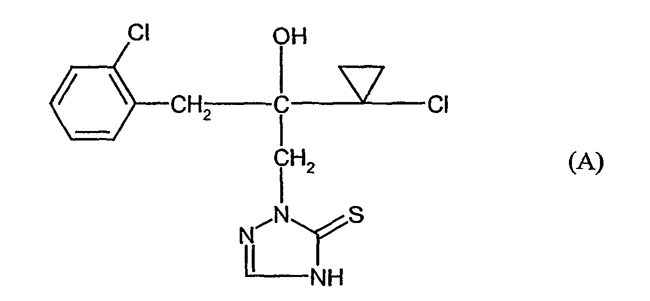 gefunden.

Weiterhin wurde gefunden, dass sich die Kristallmodifikation II des 2-[2-(1-Chlorcyclopropyl)-3-(2-chlorphenyl)-2-hydroxy-propyl]-2,4-dihydro-3H-1,2,4-triazol-3-thions der Formel (A) dadurch herstellen lässt, dass man die Kristallmodifikation I dieser Substanz in Gegenwart von
- Wasser und/oder
- einem oder mehreren aliphatischen Alkoholen mit 1 bis 10 Kohlenstoffatomen und/oder
- einem oder mehreren Dialkylketonen mit 1 bis 4 Kohlenstoffatomen in jedem Alkylteil und/oder
- einem oder mehreren Alkylcarbonsäure-alkylestern mit 1 bis 4 Kohlenstoffatomen in jedem Alkylteil
bei Temperaturen zwischen 0°C und 90°C behandelt.

Schließlich wurde gefunden, dass sich die Kristallmodifikation II des 2-[2-(1-Chlorcyclopropyl)-3-(2-chlorphenyl)-2-hydroxy-propyl]-2,4-dihydro-3H-1,2,4-triazol-3-thions der Formel (A) sehr gut zur Bekämpfung von unerwünschten Mikroorganismen, insbesondere Pilzen, verwenden lässt.

Metastabile Kristallmodifikationen weisen gegenüber den entsprechenden thermodynamisch stabilen Modifikationen generell Nachteile auf. So kann eine metastabile Modifikation den Herstellungsprozess sowie die Stabilität des Wirkstoffes oder seiner Formulierungen beim Transport oder während der Lagerung negativ beeinflussen. Zum Beispiel ist aus J. Pharm. Sci. 1969, 58, 911 bekannt, dass beim Einsatz einer thermodynamisch metastabilen Kristallform bei der Herstellung oder Lagerung eine teilweise oder vollständige Umwandlung in eine andere polymorphe Form stattfinden kann. Dies führt zu unerwünschtem Kristallwachstum (Rekristallisationen), Veränderungen in der Bioverfügbarkeit, Verbackung oder Agglomeration, wobei die Umwandlung spontan oder über einen längeren Zeitraum erfolgen kann und nicht vorhersehbar ist.

Die metastabile Modifikation I des 2-[2-(1-Chlor-cyclopropyl)-3-(2-chlorphenyl)-2-hydroxy-propyl]-2,4-dihydro-3H-1,2,4-triazol-3-thions weist ebenfalls für die Praxis ungünstige physiko-chemische Eigenschaften auf. Demgegenüber zeichnet sich die erfindungsgemäße Modifikation II dadurch aus, dass sie thermodynamisch stabil ist und sowohl ihre Herstellung als auch ihre Lagerung als Substanz oder in Form von Formulierungen, insbesondere Suspensionskonzentraten, keinerlei Probleme bereitet. Die Existenz der erfindungsgemäßen Kristallmodifikation II des Triazol-Derivates der Formel (A) ist unerwartet, denn aufgrund des vorbekannten Standes der Technik ließ sich ihr Auftreten nicht vorhersagen.

Die erfindungsgemäße Kristallmodifikation II des Triazol-Derivates der Formel (A) ist unterhalb von 90°C bei einem Druck von 1013 mbar stabil. Sie zeigt einen Schmelzpunkt von 138,3°C und kann durch Raman-Spektroskopie charakterisiert werden.

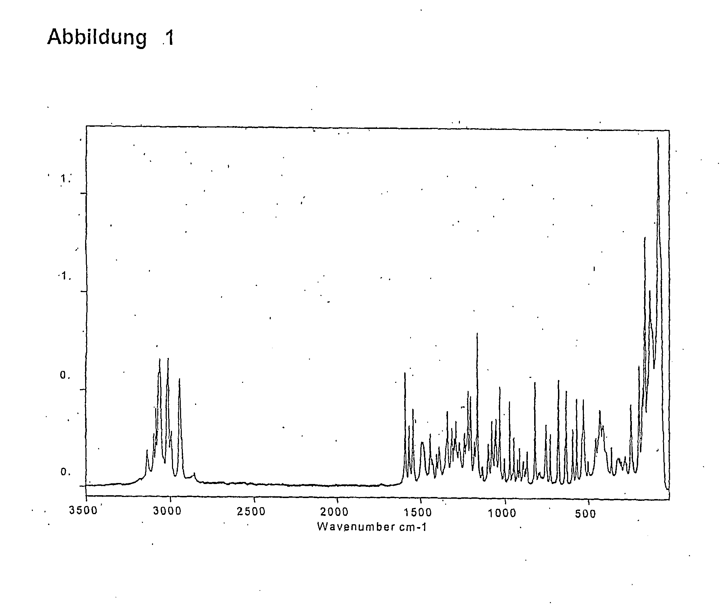

### Abbildung 1 zeigt ein Raman-Spektrum der Kristallmodifikation II des Triazol-Derivates der Formel (A). Die Werte der Peakmaxima sind in der folgenden Tabelle 1 aufgeführt.

**Tabelle 1**

| Wellenzahlen der Banden in Raman-Spektren von Kristallmodifikation II des Triazol-Derivates der Formel (A) | | | |
|---|---|---|---|
| **Wellenzahlen [cm⁻¹]** | **Wellenzahlen [cm⁻¹]** | **Wellenzahlen [cm⁻¹]** | **Wellenzahlen [cm⁻¹]** |
| 3220 | 1375 | 1101 | 876 |
| 3151 | 1351 | 1065 | 869 |
| 3063 | 1339 | 1052 | 849 |
| 3016 | 1324 | 1038 | 822 |
| 2927 | 1290 | 1032 | 796 |
| 1542 | 1220 | 1001 | 782 |
| 1476 | 1204 | 963 | 759 |
| 1455 | 1184 | 954 | 752 |
| 1445 | 1169 | 922 | 748 |
| 1424 | 1137 | 912 | 725 |
| 1407 | 1123 | 889 | 680 |

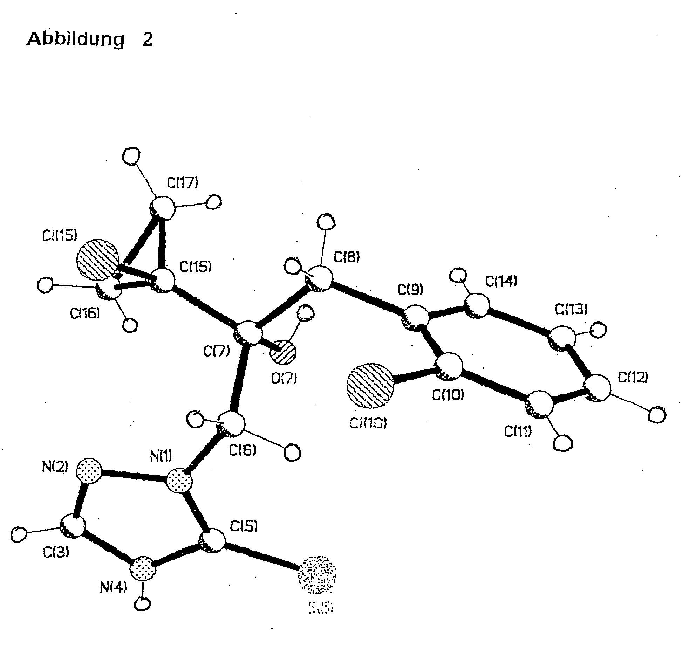

### Abbildung 2 zeigt die aus einer Röntgen-Einkristall-Strukturanalyse ermittelte Kristallstruktur der Kristallmodifikation II des Triazol-Derivates der Formel (A). Die wichtigsten Parameter, welche die Kristallstruktur eindeutig charakterisieren, sind in der folgenden Tabelle 2 aufgeführt.

**Tabelle 2**

| Bindungslängen und Winkel in Kristallen der Kristallmodifikation II des Triazol-Derivates der Formel (A) | | | | |
|---|---|---|---|---|
| **Bindung** | **Länge [Å]** | | **Bindungen** | **Winkel [°]** |
| N(1)-C(5) | 1,350 (3) | | C(5)-N(1)-N(2) | 112,8 (2) |
| N(1)-C(6) | 1,454 (3) | | N(2)-N(1)-C(6) | 120,6 (2) |
| C(3)-N(4) | 1,360 (3) | | N(2)-C(3)-N(4) | 111,9 (2) |
| S(5)-C(5) | 1,689 (2) | | N(1)-C(5)-N(4) | 103,6 (2) |
| O(7)-C(7) | 1,433 (3) | | N(4)-C(5)-S(5) | 127,8 (2) |
| C(7)-C(8) | 1,539 (3) | | O(7)-C(7)-C(6) | 104,8 (2) |
| C(9)-C(14) | 1,393 (4) | | C(6)-C(7)-C(15) | 113,6 (2) |
| C1(10)-C(10) | 1,743 (3) | | C(6)-C(7)-C(8) | 109,9 (2) |
| C(11)-C(12) | 1,384 (4) | | C(9)-C(8)-C(7) | 117,2 (2) |
| C(13)-C(14) | 1,391 (4) | | C(14)-C(9)-C(8) | 119,6 (2) |
| C(15)-C(16) | 1,490 (4) | | C(11)-C(10)-C(9) | 122,4 (2) |
| C(16)-C(17) | 1,521 (4) | | C(9)-C(10)-C1(10) | 120,1 (3) |
| N(1)-N(2) | 1,377 (3) | | C(13)-C(12)-C(11) | 119,9 (3) |
| N(2)-C(3) | 1,301 (4) | | C(13)-C(14)-C(9) | 121,9 (3) |
| N(4)-C(5) | 1,361 (3) | | C(16)-C(15)-C(7) | 123,2 (2) |
| C(6)-C(7) | 1,533 (3) | | C(16)-C(15)-C1(15) | 115,7 (2) |
| C(7)-C(15) | 1,536 (3) | | C(7)-C(15)-C1(15) | 112,2 (2) |
| C(8)-C(9) | 1,515 (3) | | C(15)-C(17)-C(16) | 59,0 (2) |
| C(9)-C(10) | 1,395 (4) | | C(5)-N(1)-C(6) | 126,6 (2) |
| C(10)-C(11) | 1,382 (4) | | C(3)-N(2)-N(1) | 103,5 (2) |
| C(12)-C(13) | 1,379 5) | | C(3)-N(4)-C(5) | 108,2 (2) |
| C1(15)-C(15) | 1,773 (3) | | N(1-C(5)-S(5) | 128,5 (2) |
| C(15)-C(17) | 1,503 (4) | | N(1)-C(6)-C(7) | 113,3 (2) |
| | | | O(7)-C(7)-C(15) | 108,9 (2) |
| | | | O(7)-C(7)-C(8) | 111,7 (2) |
| | | | C(15)-C(7)-C(8) | 108,1 (2) |
| | | | C(14)-C(9)-C(10) | 116,5 (2) |
| | | | C(10)-C(9)-C(8) | 123,9 (2) |
| | | | C(11)-C(10)-C1(10) | 117,4 (2) |
| | | | C(10)-C(11)-C(12) | 119,5 (3) |
| | | | C(12)-C(13)-C(14) | 119,8 (3) |
| | | | C(16)-C(15)-C(17) | 61,1 (2) |
| | | | C(17)-C(15)-C(7) | 120,6 (2) |
| | | | C(17)-C(15)-Cl15) | 115,1 (2) |
| | | | C(15)-C(16)-C(17) | 59,9 (2) |

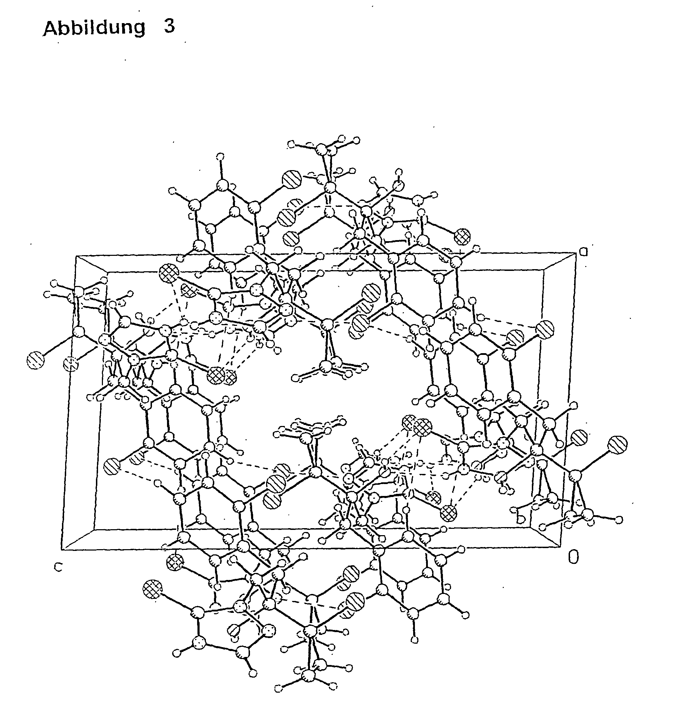

### Abbildung 3 zeigt die aus einer Röntgen-Einkristall-Strukturanalyse ermittelte Kristallpackung der Kristallmodifikation II des Triazol-Derivates der Formel (A). Die wichtigsten Parameter, welche die Kristallpackung eindeutig charakterisieren, sind in der folgenden Tabelle 3 aufgeführt.

**Tabelle 3**

| Kristallographische Daten der Kristallmodifikation II des Triazol-Derivates der Formel (A) (Kristallpackung) | | |
|---|---|---|
| **Symmetrietyp** | Monoklin | |
| **Raumgruppe** | P2₁/n | |
| **Dimensionen Volumen** | a = 9.8927(8) Å | α = 90° |
| | b = 9.5635 (8) Å | β = 92,651 (6)° |
| | c =16.4448 (10) Å | γ = 90° |
| | 1554.2(2) Å³ | |
| **Koordinate Z** | 4 | |
| **Dichte (rechnerisch)** | 1,471 Mg/m³ | |

Die bei der Herstellung der erfindungsgemäßen Substanz als Ausgangsmaterial benötigte Kristallmodifikation I des Triazol-Derivates der Formel (A) ist bekannt (vgl. WO 96-16 048). Sie weist einen Schmelzpunkt von 140,3°C auf und kann durch Raman-Spektroskopie charakterisiert werden.

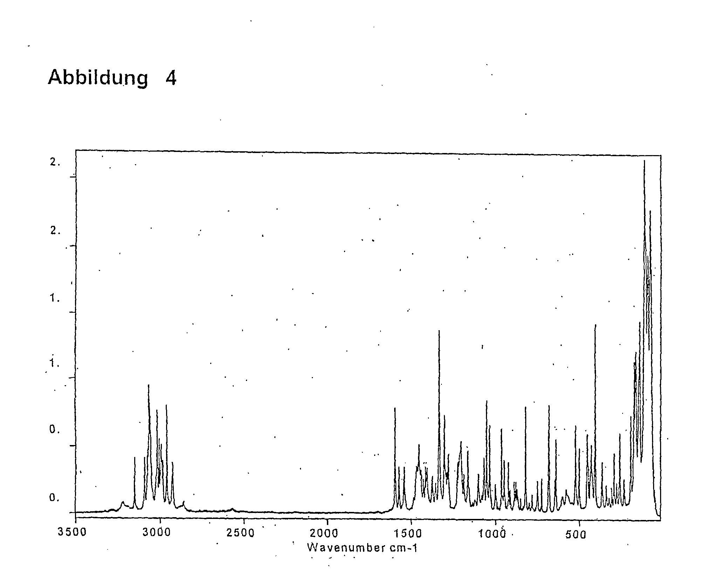

### Abbildung 4 zeigt ein Raman-Spektrum der Kristallmodifikation I des Triazol-Derivates der Formel (A). Die Werte der Peakmaxima sind in der folgenden Tabelle 4 aufgeführt.

**Tabelle 4**

| Wellenzahlen der Banden in Raman-Spektren von Kristallmodifikation I des Triazol-Derivates der Formel (A) | | | |
|---|---|---|---|
| **Wellenzahlen [cm⁻¹]** | **Wellenzahlen [cm⁻¹]** | **Wellenzahlen [cm⁻¹]** | **Wellenzahlen [cm⁻¹]** |
| 3312 | 1424 | 1133 | 947 |
| 3134 | 1406 | 1094 | 913 |
| 3070 | 1388 | 1066 | 868 |
| 3014 | 1346 | 1061 | 818 |
| 2936 | 1341 | 1053 | 779 |
| 1559 | 1291 | 1036 | 755 |
| 1488 | 1270 | 1032 | 748 |
| 1475 | 1218 | 1001 | 728 |
| 1437 | 1172 | 972 | 678 |

In der folgenden Tabelle 5 sind die Feststoffdichten der Kristallmodifikationen I und II des Triazol-Derivates der Formel (A) gegenüber gestellt.

**Tabelle 5**

| Feststoffdichten von Kristallmodifikationen | |
|---|---|
| Polymorph | Dichte [Mg/m³] |
| (Modifikation I), experimentell | 1,39 |
| (Modifikation I), berechnet aus EKS | 1,432 |
| (Modifikation II), experimentell | 1,43 |
| (Modifikation II), berechnet aus EKS | 1,471 |

Für die Bestimmung der Schmelzpunkte wurde eine DSC Pyris 1 der Fa. Perkin Elmer verwendet. Die Messungen erfolgten mit einer Heizrate von 10 K min⁻¹. Die Angaben der Schmelzpunkte beziehen sich in jedem Fall auf das Peakmaximum unter den angegebenen Bedingungen. Die Raman-Spektren der Kristallmodifikationen wurden mit einem RFS 100/S FT-Raman der Fa. Bruker erstellt (128 Scans pro Messung). Die Feststoffdichte wurde experimentell nach der Dichtebestimmungsmethode SOP 5024 mit dem Ultrapyknometer 1000 T der Fa. Quanta-Chrome bestimmt bzw. theoretisch aus der Röntgeneinkristallstrukturanalyse (EKS) ermittelt. Für die Röntgeneinkristallstrukturanalyse wurde ein P4RA-Vierkreisdiffraktometer der Fa. Siemens mit Drehanodengenerator, Graphitmonochromator, Szintillationszähler und Tieftemperaturanlage verwendet. Die Messung erfolgte mit Molybdänstrahlung einer Wellenlänge von 0,71073 (MoK_{α}).

Die zuvor angegebenen Messwerte lassen erkennen, dass sich die Kristallmodifikationen I und II des Triazol-Derivates der Formel (A) über den klar unterscheidbaren Schmelzpunkt hinaus auch über das jeweilige Raman-Spektrum sowie über die Feststoffdichte eindeutig charakterisieren lassen.

Bei der Durchführung des erfindungsgemäßen Verfahrens kommen als Verdünnungsmittel vorzugsweise Wasser, Methanol, Ethanol, 2-Propanol, Aceton, 2-Butanon und Ethylacetat in Frage. Dabei können die Solventien sowohl einzeln als auch in Form von Gemischen eingesetzt werden.

Die Temperaturen können bei der Durchführung des erfindungsgemäßen Verfahrens in einem bestimmten Bereich variiert werden. Im Allgemeinen arbeitet man bei Temperaturen zwischen 0°C und 90°C, vorzugsweise bei Temperaturen zwischen 0°C und 80°C, besonders bevorzugt zwischen 50°C und 80°C.

Bei der Durchführung des erfindungsgemäßen Verfahrens arbeitet man im Allgemeinen unter Atmosphärendruck. Es ist aber auch möglich, unter erhöhtem Druck zu arbeiten.

Bei der Durchführung des erfindungsgemäßen Verfahrens geht man im Allgemeinen so vor, dass man die jeweils gewünschte Menge an Kristallmodifikation I des Triazol-Derivates der Formel (A) in dem jeweiligen Verdünnungsmittel suspendiert oder löst und dann bei der jeweils gewünschten Temperatur bis zur Umwandlung in die Kristallmodifikation II rührt. Die Reaktionsdauer hängt dabei sowohl von der Reaktionstemperatur als auch vom Verdünnungsmittel ab. Außerdem hängt die Umwandlungsgeschwindigkeit davon ab, ob Impfkristalle der Kristallmodifikation II vorhanden sind. Arbeitet man bei höheren Temperaturen, so vollzieht sich die Umwandlung schneller als bei tiefen Temperaturen. Verwendet man ein Solvens, in dem sich die Kristallmodifikation I des Triazol-Derivates der Formel (A) vollständig löst, so verläuft die Umwandlung in die Kristallmodifikation II schneller als beim Einsatz von Suspensionen, in denen das Ausgangsprodukt gar nicht oder nur zu einem geringen Teil gelöst ist. Ebenso wird die Umwandlung von der Kristallmodifikation I in die Kristallmodifikation II durch die Anwesenheit von Impfkristallen der Kristallmodifikation II beschleunigt.

Im Allgemeinen kann die Umwandlung von Kristallen der Modifikation I in die Modifikation II bei erhöhter Temperatur durch Kühlungskristallisation zur Raumtemperatur ohne die Verwendung von Impfkristallen direkt erzielt werden. Für die Umwandlung einer Suspension von Kristallen der Modifikation I in die Modifikation II wird ohne die Verwendung von Impfkristallen ein Zeitraum von 7 bis 14 Tagen benötigt. Werden bei der Umwandlung einer Suspension von Kristallen der Modifikation I in die Modifikation II hingegen Impfkristalle der Modifikation II hinzugefügt, so ist im Allgemeinen eine Behandlungsdauer von 24 bis 48 Stunden ausreichend, um eine quantitative Umwandlung in die Kristallmodifikation II zu erreichen. Eine Verlängerung der Behandlungsdauer ist jeweils möglich, ohne dass sich dabei wieder Kristallmodifikation I bildet.

Die Isolierung der Kristalle der Modifikation II erfolgt jeweils nach üblichen Methoden. Liegt eine Suspension vor, so geht man im Allgemeinen so vor, dass man die Kristalle der Modifikation II abfiltriert und trocknet.

Wenn bei der Durchführung des erfindungsgemäßen Verfahrens die Umwandlung in die Kristallmodifikation II nicht quantitativ vorgenommen wird, so erhält man Gemische von Kristallen der Modifikationen I und II. Da die Kristallmodifikation I jedoch thermodynamisch weniger stabil ist als die Modifikation II, sollte der erfindungsgemäße Wirkstoff jedoch nur einen geringen Anteil an Kristallmodifikation I enthalten. In den erfindungsgemäßen Produkten sind im Allgemeinen weniger als 10 Gew.-% an Kristallmodifikation I, bevorzugt weniger als 5 Gew.-%, besonders bevorzugt weniger als 2 Gew.-% an Kristallmodifikation I vorhanden.

Aufgrund ihrer thermodynamischen Stabilität eignet sich die erfindungsgemäße Kristallmodifikation II des Triazol-Derivates der Formel (A) sehr gut zur Herstellung von Formulierungen, und zwar auch dann, wenn der Wirkstoff nach der Herstellung der Formulierung nicht mehr in kristalliner Form, sondern in Lösung vorliegt. Vorteilhaft ist insbesondere, dass die Kristallmodifikation II des Triazol-Derivates der Formel (A) jeweils quantitativ in die gewünschte Formulierung überführt wird. Das Risiko einer Fehldosierung bedingt durch Agglomerisation und/oder Sedimentation wird dadurch entscheidend herabgesetzt.

Der erfindungsgemäße Wirkstoff, also das Triazol-Derivat der Formel (A) in der Kristallmodifikation II, weist eine hervorragende mikrobizide Wirkung auf und kann zur Bekämpfung von unerwünschten Mikroorganismen, wie Fungi und Bakterien, im Pflanzenschutz und im Materialschutz eingesetzt werden.

Mit dem erfindungsgemäßen Wirkstoff können Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die Behandlung der Pflanzen und Pflanzenteile mit dem erfindungsgemäßen Wirkstoff erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Im Materialschutz lässt sich der erfindungsgemäße Stoff zum Schutz von technischen Materialien gegen Befall und Zerstörung durch unerwünschte Mikroorganismen einsetzen.

Die Kristallmodifikation des Triazol-Derivates der Formel (A) kann in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen des Wirkstoffes mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im Allgemeinen zwischen 0,1 und 95 Gewichtsprozent Wirkstoff, vorzugsweise zwischen 0,5 und 90 %.

Der erfindungsgemäße Wirkstoff kann als solcher oder in seinen Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

### Als Mischpartner kommen zum Beispiel folgende Verbindungen in Frage:

### Fungizide:

Aldimorph, Ampropylfos, Ampropylfos-Kalium, Andoprim, Anilazin, Azaconazol, Azoxystrobin,
Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat,
Calciumpolysulfid, Carpropamid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Ediphenphos, Epoxiconazol, Etaconazol, Ethirimol, Etridiazol,
Famoxadon, Fenapanil, Fenarimol, Fenbuconazol, Fenfuram, Fenhexamid, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox, Fluoxastrobin,
Guazatin,
Hexachlorobenzol, Hexaconazol, Hymexazol,
Imazalil, Imibenconazol, Iminoctadin, Iminoctadinealbesilat, Iminoctadinetriacetat, Iodocarb, Ipconazol, Iprobenfos (IBP), Iprodione, Iprovalicarb, Irumamycin, Isoprothiolan, Isovaledione,
Kasugamycin, Kresoxim-methyl, Kupfer-Zubereitungen, wie: Kupferhydroxid, Kupfemaphthenat, Kupferoxychlorid, Kupfersulfat, Kupferoxid, Oxin-Kupfer und Bordeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol,
Ofurace, Oxadixyl, Oxamocarb, Oxolinicacid, Oxycarboxim, Oxyfenthiin,
Paclobutrazol, Pefurazoat, Penconazol, Pencycuron, Phosdiphen, Picoxystrobin, Pimaricin, Piperalin, Polyoxin, Polyoxorim, Probenazol, Prochloraz, Procymidon, Propamocarb, Propanosine-Natrium, Propiconazol, Propineb, Pyraclostrobin, Pyrazophos, Pyrifenox, Pyrimethanil, Pyroquilon, Pyroxyfur,
Quinconazol, Quintozen (PCNB), Quinoxyfen
Schwefel und Schwefel-Zubereitungen, Spiroxamine
Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Trifloxystrobin, Triflumizol, Triforin, Triticonazol,
Uniconazol,
Validamycin A, Vinclozolin, Viniconazol,
Zarilamid, Zineb, Ziram sowie
Dagger G,
OK-8705,
OK-8801,
α-(1,1-Dimethylethyl)-β-(2-phenoxyethyl)-1H-1,2,4-triazol-1-ethanol,
α-(2,4-Dichlorphenyl)-β-fluor-propyl-1H-1,2,4-triazol-1-ethanol,
α-(2,4-Dichlorphenyl)-β-methoxy-a-methyl-1H-1,2,4-triazol-1-ethanol,
α-(5-Methyl-1,3-dioxan-5-yl)-β-[[4-(trifluormethyl)-phenyl]-methylen]-1H-1,2,4-triazol-1-ethanol,
(5RS,6RS)-6-Hydroxy-2,2,7,7-tetramethyl-5-(1H-1,2,4-triazol-1-yl)-3-octanon,
(E)-α-(Methoxyimino)-N-methyl-2-phenoxy-phenylacetamid,
1-(2,4-Dichlorphenyl)-2-(1H-1,2,4-triazol-1-yl)-ethanon-O-(phenylmethyl)-oxim,
1-(2-Methyl-1-naphthalenyl)-1H-pyrrol-2,5-dion,
1-(3,5-Dichlorphenyl)-3-(2-propenyl)-2,5-pyrrolidindion,
1-[(Diiodmethyl)-sulfonyl]-4-methyl-benzol,
1-[[2-(2,4-Dichlorphenyl)-1,3-dioxolan-2-yl]-methyl]-1H-imidazol,
1-[[2-(4-Chlorphenyl)-3-phenyloxiranyl]-methyl]-1H-1,2,4-triazol,
1-[1-[2-[(2,4-Dichlorphenyl)-methoxy]-phenyl]-ethenyl]-1H-imidazol,
1-Methyl-5-nonyl-2-(phenylmethyl)-3-pyrrolidinol,
2',6'-Dibrom-2-methyl-4'-trifluormethoxy-4-trifluormethyl-1,3-thiazol-5-carboxanilid,
2,6-Dichlor-5-(methylthio)-4-pyrimidinyl-thiocyanat,
2,6-Dichlor-N-(4-trifluormethylbenzyl)-benzamid,
2,6-Dichlor-N-[[4-(trifluormethyl)-phenyl]-methyl]-benzamid,
2-(2,3,3-Triiod-2-propenyl)-2H-tetrazol,
2-[(1-Methylethyl)-sulfonyl]-5-(trichlormethyl)-1,3,4-thiadiazol,
2-[[6-Deoxy-4-O-(4-O-methyl-β-D-glycopyranosyl)-α-D-glucopyranosyl]-amino]-4-methoxy-1H-pyrrolo[2,3-d]pyrimidin-5-carbonitril,
2-Aminobutan,
2-Brom-2-(brommethyl)-pentandinitril,
2-Chlor-N-(2,3-dihydro-1,1,3-trimethyl-1H-inden-4-yl)-3-pyridincarboxamid,
2-Chlor-N-(2,6-dimethylphenyl)-N-(isothiocyanatomethyl)-acetamid,
2-Phenylphenol(OPP),
3,4-Dichlor-1-[4-(difluormethoxy)-phenyl]-1H-pyrrol-2,5-dion,
3,5-Dichlor-N-[cyan[(1-methyl-2-propynyl)-oxy]-methyl]-benzamid,
3-(1,1-Dimethylpropyl-1-oxo-1H-inden-2-carbonitril,
3-[2-(4-Chlorphenyl)-5-ethoxy-3-isoxazolidinyl]-pyridin,
4-Chlor-2-cyan-N,N-dimethyl-5-(4-methylphenyl)-1H-imidazol-1-sulfonamid,
4-Methyl-tetrazolo[1,5-a]quinazolin-5(4H)-on,
8-Hydroxychinolinsulfat,
9H-Xanthen-9-carbonsäure-2-[(phenylamino)-carbonyl]-hydrazid,
bis-(1-Methylethyl)-3-methyl-4-[(3-methylbenzoyl)-oxy]-2,5-thiophendicarboxylat,
cis-1-(4-Chlorphenyl)-2-(1H-1,2,4-triazol-1-yl)-cycloheptanol,
cis-4-[3-[4-(1,1-Dimethylpropyl)-phenyl-2-methylpropyl]-2,6-dimethyl-morpholin-hydrochlorid,
Ethyl-[(4-chlorphenyl)-azo]-cyanoacetat,
Kaliumhydrogencarbonat,
Methantetrathiol-Natriumsalz,
Methyl-1-(2,3-dihydro-2,2-dimethyl-1H-inden-1-yl)-1H-imidazol-5-carboxylat,
Methyl-N-(2,6-dimethylphenyl)-N-(5-isoxazolylcarbonyl)-DL-aianinat,
Methyl-N-(chloracetyl)-N-(2,6-dimethylphenyl)-DL-alaninat,
N-(2,6-Dimethylphenyl)-2-methoxy-N-(tetrahydro-2-oxo-3-furanyl)-acetamid,
N-(2,6-Dimethylphenyl)-2-methoxy-N-(tetrahydro-2-oxo-3-thienyl)-acetamid,
N-(2-Chlor-4-nitrophenyl)-4-methyl-3-nitro-benzolsulfonamid,
N-(4-Cyclohexylphenyl)-1,4,5,6-tetrahydro-2-pyrimidinamin,
N-(4-Hexylphenyl)-1,4,5,6-tetrahydro-2-pyrimidinamin,
N-(5-Chlor-2-methylphenyl)-2-methoxy-N-(2-oxo-3-oxazolidinyl)-acetamid,
N-(6-Methoxy)-3-pyridinyl)-cyclopropancarboxamid,
N-[2,2,2-Trichlor-1-[(chloracetyl)-amino]-ethyl]-benzamid,
N-[3-Chlor-4,5-bis-(2-propinyloxy)-phenyl]-N'-methoxy-methanimidamid,
N-Formyl-N-hydroxy-DL-alanin -Natriumsalz,
O,O-Diethyl-[2-(dipropylamino)-2-oxoethyl]-ethylphosphoramidothioat,
O-Methyl-S-phenyl-phenylpropylphosphoramidothioate,
S-Methyl-1,2,3-benzothiadiazol-7-carbothioat,
spiro[2H]-1-Benzopyran-2,1'(3'H)-isobenzofuran]-3'-on,
4-[(3,4-Dimethoxyphenyl)-3-(4-fluorphenyl)-acryloyl]-morpholin

### Bakterizide:

Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Acephate, Acetamiprid, Acrinathrin, Alanycarb, Aldicarb, Aldoxycarb, Alpha-cypermethrin, Alphamethrin, Amitraz, Avermectin, AZ 60541, Azadirachtin, Azamethiphos, Azinphos A, Azinphos M, Azocyclotin,
Bacillus popilliae, Bacillus sphaericus, Bacillus subtilis, Bacillus thuringiensis, Baculoviren, Beauveria bassiana, Beauveria tenella, Bendiocarb, Benfuracarb, Bensultap, Benzoxmiate, Betacyfluthrin, Bifenazate, Bifenthrin, Bioethanomethrin, Biopermethrin, Bistrifluron, BPMC, Bromophos A, Bufencarb, Buprofezin, Butathiofos, Butocarboxim, Butylpyridaben,
Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, Chlorpyrifos, Chlorpyrifos M, Chlovaporthrin, Chromafenozide, Cis-Resmethrin, Cispermethrin, Clocythrin, Cloethocarb, Clofentezine, Clothianidine, Cyanophos, Cycloprene, Cycloprothrin, Cyfluthrin, Cyhalothrin, Cyhexatin, Cypermethrin, Cyromazine,
Deltamethrin, Demeton M, Demeton S, Demeton-S-methyl, Diafenthiuron, Diazinon, Dichlorvos, Dicofol, Diflubenzuron, Dimethoat, Dimethylvinphos, Diofenolan, Disulfoton, Docusat-sodium, Dofenapyn,
Eflusilanate, Emamectin, Empenthrin, Endosulfan, Entomopfthora spp., Esfenvalerate, Ethiofencarb, Ethion, Ethoprophos, Etofenprox, Etoxazole, Etrimfos, Fenamiphos, Fenazaquin, Fenbutatin oxide, Fenitrothion, Fenothiocarb, Fenoxacrim, Fenoxycarb, Fenpropathrin, Fenpyrad, Fenpyrithrin, Fenpyroximate, Fenvalerate, Fipronil, Fluazuron, Flubrocythrinate, Flucycloxuron, Flucythrinate, Flufenoxuron, Flumethrin, Flutenzine, Fluvalinate, Fonophos, Fosmethilan, Fosthiazate, Fubfenprox, Furathiocarb,
Granuloseviren
Halofenozide, HCH, Heptenophos, Hexaflumuron, Hexythiazox, Hydroprene,
Imidacloprid, Indoxacarb, Isazofos, Isofenphos, Isoxathion, Ivermectin,
Kempolyederviren
Lambda-cyhalothrin, Lufenuron
Malathion, Mecarbam, Metaldehyd, Methamidophos, Metharhizium anisopliae, Metharhizium flavoviride, Methidathion, Methiocarb, Methoprene, Methomyl, Methoxyfenozide, Metolcarb, Metoxadiazone, Mevinphos, Milbemectin, Milbemycin, Monocrotophos,
Naled, Nitenpyram, Nithiazine, Novaluron
Omethoat, Oxamyl, Oxydemethon M
Paecilomyces fumosoroseus, Parathion A, Parathion M, Permethrin, Phenthoat, Phorat, Phosalone, Phosmet, Phosphamidon, Phoxim, Pirimicarb, Pirimiphos A, Pirimiphos M, Profenofos, Promecarb, Propargite, Propoxur, Prothiofos, Prothoat, Pymetrozine, Pyraclofos, Pyresmethrin, Pyrethrum, Pyridaben, Pyridathion, Pyrimidifen, Pyriproxyfen,
Quinalphos,
Ribavirin
Salithion, Sebufos, Silafluofen, Spinosad, Spirodiclofen, Sulfotep, Sulprofos,
Tau-fluvalinate, Tebufenozide, Tebufenpyrad, Tebupirimiphos, Teflubenzuron, Tefluthrin, Temephos, Temivinphos, Terbufos, Tetrachlorvinphos, Tetradifon, Thetacypermethrin, Thiacloprid, Thiamethoxam, Thiapronil, Thiatriphos, Thiocyclam hydrogen oxalate, Thiodicarb, Thiofanox, Thuringiensin, Tralocythrin, Tralomethrin, Triarathene, Triazamate, Triazophos, Triazuron, Trichlophenidine, Trichlorfon, Triflumuron, Trimethacarb,
Vamidothion, Vaniliprole, Verticillium lecanii
YI 5302
Zeta-cypermethrin, Zolaprofos
(1R-cis)-[5-(Phenylmethyl)-3-furanyl]-methyl-3-[(dihydro-2-oxo-3((2H)-furanyliden)-methyl]-2,2-dimethylcyclopropancarboxylat
(3-Phenoxyphenyl)-methyl-2,2,3,3-tetramethylcyclopropanecarboxylat
1-[(2-Chlor-5-thiazolyl)methyl]tetrahydro-3,5-dimethyl-N-nitro-1,3,5-triazin-2(1H)-imin
2-(2-Chlor-6-fluorphenyl)-4-[4-(1,1-dimethylethyl)phenyl]-4,5-dihydro-oxazol
2-(Acetlyoxy)-3-dodecyl-1,4-naphthalindion
2-Chlor-N-[[[4-(1-phenylethoxy)-phenyl]-amino]-carbonyl]-benzamid
2-Chlor-N-[[[4-(2,2-dichlor-1,1-difluorethoxy)-phenyl]-amino]-carbonyl]-benzamid
3-Methylphenyl-propylcarbamat
4-[4-(4-Ethoxyphenyl)-4-methylpentyl]-1-fluor-2-phenoxy-benzol
4-Chlor-2-(1,1-dimethylethyl)-5-[[2-(2,6-dimethyl-4-phenoxyphenoxy)ethyl]thio]-3(2H)-pyridazinon
4-Chlor-2-(2-chlor-2-methylpropyl)-5-[(6-iod-3-pyridinyl)methoxy]-3(2H)-pyridazinon
4-Chlor-5-[(6-chlor-3-pyridinyl)methoxy]-2-(3,4-dichlorphenyl)-3(2H)-pyridazinon
Bacillus thuringiensis strain EG-2348
Benzoesäure [2-benzoyl-1-(1,1-dimethylethyl)-hydrazid
Butansäure 2,2-dimethyl-3-(2,4-dichlorphenyl)-2-oxo-1-oxaspiro[4.5]dec-3-en-4-yl-ester
[3-[(6-Chlor-3-pyridinyl)methyl]-2-thiazolidinyliden]-cyanamid
Dihydro-2-(nitromethylen)-2H-1,3-thiazine-3(4H)-carboxaldehyd
Ethyl-[2-[[1,6-dihydro-6-oxo-1-(phenylmethyl)-4-pyridazinyl]oxy]ethyl]-carbamat
N-(3,4,4-Trifluor-1-oxo-3-butenyl)-glycin
N-(4-Chlorphenyl)-3-[4-(difluormethoxy)phenyl]-4,5-dihydro-4-phenyl-1H-pyrazol-1-carboxamid
N-[(2-Chlor-5-thiazolyl)methyl]-N'-methyl-N"-nitro-guanidin
N-Methyl-N'-(1-methyl-2-propenyl)-1,2-hydrazindicarbothioamid
N-Methyl-N'-2-propenyl-1,2-hydrazindicarbothioamid
O,O-Diethyl-[2-(dipropylamino)-2-oxoethyl]-ethylphosphoramidothioat
N-Cyanomethyl-4-trifluormethyl-nicotinamid
3,5-Dichlor-1-(3,3-dichlor-2-propenyloxy)-4-[3-(5-trifluormethylpyridin-2-yloxy)-propoxy]-benzol

Auch eine Mischung mit anderen bekannten Wirkstoffen, wie Herbiziden oder mit Düngemitteln und Wachstumsregulatoren ist möglich.

Darüber hinaus weist der erfindungsgemäße Wirkstoff auch sehr gute antimykotische Wirkungen auf. Er besitzt ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Der Wirkstoff kann als solcher, in Form seiner Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, den Wirkstoff nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Wie bereits oben erwähnt, können mit dem erfindungsgemäßen Wirkstoff alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetic Modified Organisms) und deren Teile behandelt. Der Begriff "Teile" bzw. "Teile von Pflanzen" oder "Pflanzenteile" wurde oben erläutert.

Besonders bevorzugt werden Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Je nach Pflanzenarten bzw. Pflanzensorten, deren Standort und Wachstumsbedingungen (Böden, Klima, Vegetationsperiode, Ernährung) können durch die Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung des erfindungsgemäß verwendbaren Stoffes, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugt zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte.

Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Raps sowie Obstpflanzen (mit den Früchten Äpfel, Birnen, Zitrusfrüchten und Weintrauben) erwähnt, wobei Mais, Soja, Kartoffel, Baumwolle und Raps besonders hervorgehoben werden. Als Eigenschaften ("Traits") werden besonders hervorgehoben die erhöhte Abwehr der Pflanzen gegen Insekten durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft mit dem erfindungsgemäßen Wirkstoff oder dessen Mischungen behandelt werden.

Die Herstellung der Kristallmodifikation II des Triazol-Derivates der Formel (A) wird durch die folgenden Beispiele veranschaulicht.

### Herstellungsbeispiele

### Beispiel 1

5 g des Triazol-Derivates der Formel (A) in der Kristallmodifikation I werden in 50 g Methanol suspendiert. Die Suspension wird so lange auf 60°C erwärmt und gerührt, bis sich die Kristalle der Modifikation I vollständig gelöst haben. Anschließend wird auf Raumtemperatur abgekühlt. Dabei fällt ein kristallines Produkt aus, das abfiltriert und bei Temperaturen unterhalb von 60°C getrocknet wird. Man erhält auf diese Weise 4 g an Triazol-Derivat der Formel (A) in der Kristallmodifikation II. Das Produkt zeigt im Raman-Spektrum Peakmaxima bei den in Tabelle 1 angegebenen Wellenzahlen.
Schmelzpunkt: 140,0°C (Peakmaximum)

### Beispiel 2

5 g des Triazol-Derivates der Formel (A) in der Kristallmodifikation I werden in 40 g Aceton suspendiert. Die Suspension wird so lange auf 50°C erwärmt und gerührt, bis sich die Kristalle der Modifikation I vollständig gelöst haben. Anschließend wird auf Raumtemperatur abgekühlt. Dabei fällt ein kristallines Produkt aus, das abfiltriert und bei Temperaturen unterhalb von 60°C getrocknet wird. Man erhält auf diese Weise 3 g an Triazol-Derivat der Formel (A) in der Kristallmodifikation II. Das Produkt zeigt im Raman-Spektrum Peakmaxima bei den in Tabelle 1 angegebenen Wellenzahlen.
Schmelzpunkt: 138,6°C (Peakmaximum)

### Beispiel 3

5 g des Triazol-Derivates der Formel (A) in der Kristallmodifikation I werden in 40 g Ethylacetat suspendiert. Die Suspension wird so lange auf 70°C erwärmt und gerührt, bis sich die Kristalle der Modifikation I vollständig gelöst haben. Anschließend wird auf Raumtemperatur abgekühlt. Dabei fällt ein kristallines Produkt aus, das abfiltriert und bei Temperaturen unterhalb von 60°C getrocknet wird. Man erhält auf diese Weise 3 g an Triazol-Derivat der Formel (A) in der Kristallmodifikation II. Das Produkt zeigt im Raman-Spektrum Peakmaxima bei den in Tabelle 1 angegebenen Wellenzahlen.
Schmelzpunkt: 138,7°C (Peakmaximum)

### Beispiel 4

5 g des Triazol-Derivates der Formel (A) in der Kristallmodifikation I werden in 100 g destilliertem Wasser suspendiert. Die Suspension wird 2 Wochen bei 80°C gerührt. Danach wird das enthaltene kristalline Produkt abfiltriert und bei Temperaturen unterhalb von 60°C getrocknet. Man erhält auf diese Weise 4 g an Triazol-Derivat der Formel (A) in der Kristallmodifikation II. Das Produkt zeigt im Raman-Spektrum Peakmaxima bei den in Tabelle 1 angegebenen Wellenzahlen.
Schmelzpunkt: 138,4°C (Peakmaximum)

### Vergleichsbeispiel A

Ein Gemisch aus 3,12 g (10 mmol) 2-(1-Chlor-cyclopropyl)-1-(2-chlorphenyl)-3-(1,2,4-triazol-1-yl)-propan-2-ol und 45 ml absolutem Tetrahydrofuran wird bei -20°C mit 8,4 ml (21 mmol) n-Butyl-lithium in Hexan versetzt und 30 Minuten bei 0°C gerührt. Danach wird das Reaktionsgemisch auf -70°C abgekühlt, mit 0,32 g (10 mMol) Schwefel-Pulver versetzt und 30 Minuten bei -70°C gerührt. Es wird auf -10°C erwärmt, mit Eiswasser versetzt und durch Zugabe von verdünnter Schwefelsäure auf einen pH-Wert von 5 eingestellt. Man extrahiert mehrfach mit Essigsäureethylester, trocknet die vereinigten organischen Phasen über Natriumsulfat und engt unter vermindertem Druck ein. Man erhält auf diese Weise 3,2 g (93 % der Theorie) an 2-(1-Chlor-cyclopropyl)-1-(2-chlorphenyl)-3-(5-mercapto-1,2,4-triazol-1-yl)-propan-2-ol in der Kristallmodifikation I. Das Produkt zeigt im Raman-Spektrum Peakmaxima bei den in Tabelle 4 angegebenen Wellenzahlen.
Schmelzpunkt: 139,3°C

## Patentansprüche

1. Kristallmodifikation II des 2-[2-(1-Chlor-cyclopropyl)-3-(2-chlorphenyl)-2-hydroxy-propyl]-2,4-dihydro-3H-1,2,4-triazol-3-thions der Formel 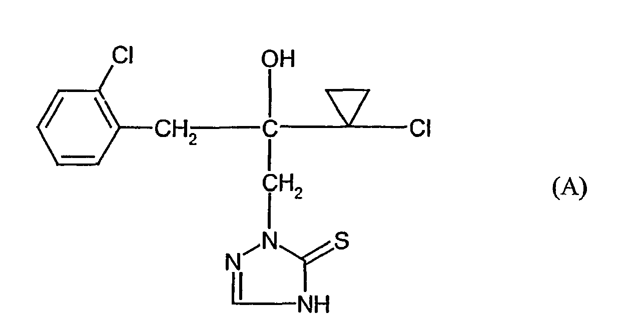 **gekennzeichnet durch**
a) Peakmaxima im Raman-Spektrum bei den folgenden Wellenzahlen [in cm⁻¹]
| | | | |
|---|---|---|---|
| 3220 | 1375 | 1101 | 876 |
| 3151 | 1351 | 1065 | 869 |
| 3063 | 1339 | 1052 | 849 |
| 3016 | 1324 | 1038 | 822 |
| 2927 | 1290 | 1032 | 796 |
| 1542 | 1220 | 1001 | 782 |
| 1476 | 1204 | 963 | 759 |
| 1455 | 1184 | 954 | 752 |
| 1445 | 1169 | 922 | 748 |
| 1424 | 1137 | 912 | 725 |
| 1407 | 1123 | 889 | 680 |
b) die folgenden Bindungslängen [in Å] und Bindungswinkel [in °]
| **Bindung** | **Länge [Å]** | | **Bindungen** | **Winkel [°]** |
|---|---|---|---|---|
| N(1)-C(5) | 1,350 (3) | | C(5)-N(1)-N(2) | 112,8 (2) |
| N(1)-C(6) | 1,454 (3) | | N(2)-N(1)-C(6) | 120,6 (2) |
| C(3)-N(4) | 1,360 (3) | | N(2)-C(3)-N(4) | 111,9 (2) |
| S(5)-C(5) | 1,689 (2) | | N(1)-C(5)-N(4) | 103,6 (2) |
| O(7)-C(7) | 1,433 (3) | | N(4)-C(5)-S(5) | 127,8 (2) |
| C(7)-C(8) | 1,539 (3) | | O(7)-C(7)-C(6) | 104,8 (2) |
| C(9)-C(14) | 1,393 (4) | | C(6)-C(7)-C(15) | 113,6 (2) |
| C1(10)-C(10) | 1,743 (3) | | C(6)-C(7)-C(8) | 109,9 (2) |
| C(11)-C(12) | 1,384 (4) | | C(9)-C(8)-C(7) | 117,2 (2) |
| C(13)-C(14) | 1,391 (4) | | C(14)-C(9)-C(8) | 119,6 (2) |
| C(15)-C(16) | 1,490 (4) | | C(11)-C(10)-C(9) | 122,4 (2) |
| C(16)-C(17) | 1,521 (4) | | C(9)-C(10)-C1(10) | 120,1 (3) |
| N(1)-N(2) | 1,377 (3) | | C(13)-C(12)-C(11) | 119,9 (3) |
| N(2)-C(3) | 1,301 (4) | | C(13)-C(14)-C(9) | 121,9 (3) |
| N(4)-C(5) | 1,361 (3) | | C(16)-C(15)-C(7) | 123,2 (2) |
| C(6)-C(7) | 1,533 (3) | | C(16)-C(15)-C1(15) | 115,7 (2) |
| C(7)-C(15) | 1,536 (3) | | C(7)-C(15)-C1(15) | 112,2 (2) |
| C(8)-C(9) | 1,515 (3) | | C(15)-C(17)-C(16) | 59,0 (2) |
| C(9)-C(10) | 1,395 (4) | | C(5)-N(1)-C(6) | 126,6 (2) |
| C(10)-C(11) | 1,382 (4) | | C(3)-N(2)-N(1) | 103,5 (2) |
| C(12)-C(13) | 1,379 (5) | | C(3)-N(4)-C(5) | 108,2 (2) |
| C1(15)-C(15) | 1,773 (3) | | N(1)-C(5)-S(5) | 128,5 (2) |
| C(15)-C(17) | 1,503 (4) | | N(1)-C(6)-C(7) | 113,3 (2) |
| | | | O(7)-C(7)-C(15) | 108,9 (2) |
| | | | O(7)-C(7)-C(8) | 111,7 (2) |
| | | | C(15)-C(7)-C(8) | 108,1 (2) |
| | | | C(14)-C(9)-C(10) | 116,5 (2) |
| | | | C(10)-C(9)-C(8) | 123,9 (2) |
| | | | C(11)-C(10)-C1(10) | 117,4 (2) |
| | | | C(10)-C(11)-C(12) | 119,5 (3) |
| | | | C(12)-C(13)-C(14) | 119,8 (3) |
| | | | C(16)-C(15)-C(17) | 61,1 (2) |
| | | | C(17)-C(15)-C(7) | 120,6 (2) |
| | | | C(17)-C(15)-Cl15) | 115,1 (2) |
| | | | C(15)-C(16)-C(17) | 59,9 (2) |
c) eine Elementarzelle mit folgenden Dimensionen
| | |
|---|---|
| a = 9.8927(8) Å | α = 90° |
| b = 9.5635 (8) Å | β = 92,651 (6)° |
| c = 16.4448 (10) Å | γ = 90° |
d) einem Schmelzpunkt von 138,3°C
und
e) einer Feststoffdichte von 1,471 Mg/m³.

2. Verfahren zur Herstellung der Kristallmodifikation II des Triazol-Derivates der Formel (A) gemäß Anspruch 1, **dadurch gekennzeichnet, dass** man die Kristallmodifikation I dieser Substanz in Gegenwart von
• Wasser und/oder
• einem oder mehreren aliphatischen Alkoholen mit 1 bis 10 Kohlenstoffatomen und/oder
• einem oder mehreren Dialkylketonen mit 1 bis 4 Kohlenstoffatomen in jedem Alkylteil und/oder
• einem oder mehreren Alkylcarbonsäure-alkylestern mit 1 bis 4 Kohlenstoffatomen in jedem Alkylteil
bei Temperaturen zwischen 0°C und 90°C behandelt.

3. Mikrobizide Mittel, **gekennzeichnet durch** einen Gehalt an Triazol-Derivat der Formel (A) gemäß Anspruch 1 in der Kristallmodifikation II neben Streckmitteln und/oder oberflächenaktiven Stoffen.

4. Verwendung von Kristallmodifikation II des Triazol-Derivates der Formel (A) gemäß Anspruch 1 zur Bekämpfung von unerwünschten Mikroorganismen.

5. Verfahren zur Bekämpfung von unerwünschten Mikroorganismen, **dadurch gekennzeichnet, dass** man Kristallmodifikation II des Triazol-Derivates der Formel (A) gemäß Anspruch 1 auf die Mikroorganismen und/oder deren Lebensraum ausbringt.

6. Verfahren zur Herstellung von mikrobiziden Mitteln, **dadurch gekennzeichnet, dass** man Kristallmodifikation II des Triazol-Derivates der Formel (A) gemäß Anspruch 1 mit Streckmitteln und/oder oberflächenaktiven Stoffen vermischt.

## Claims

1. Crystal form II of 2-[2-(1-chlorocyclopropyl)-3-(2-chlorophenyl)-2-hydroxypropyl]-2,4-dihydro-3H-1,2,4-triazole-3-thione of the formula 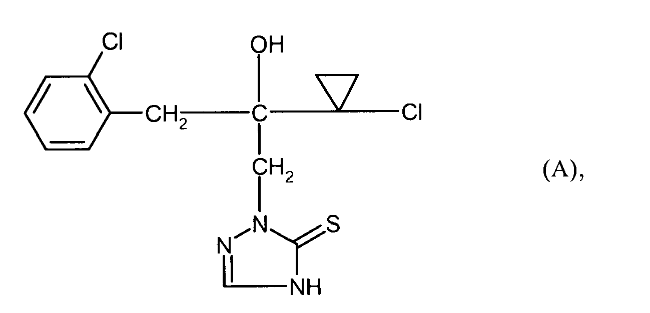 **characterized by**
a) peak maxima in the Raman spectrum at the following wave numbers [in cm⁻¹]
| | | | |
|---|---|---|---|
| 3220 | 1375 | 1101 | 876 |
| 3151 | 1351 | 1065 | 869 |
| 3063 | 1339 | 1052 | 849 |
| 3016 | 1324 | 1038 | 822 |
| 2927 | 1290 | 1032 | 796 |
| 1542 | 1220 | 1001 | 782 |
| 1476 | 1204 | 963 | 759 |
| 1455 | 1184 | 954 | 752 |
| 1445 | 1169 | 922 | 748 |
| 1424 | 1137 | 912 | 725 |
| 1407 | 1123 | 889 | 680 |
b) the following bond lengths [in A] and bond angles [in °]
| **Bond** | **Length [Å]** | | **Bonds** | **Angle [°]** |
|---|---|---|---|---|
| N(1)-C(5) | 1.350 (3) | | C(5)-N(1)-N(2) | 112.8 (2) |
| N(1)-C(6) | 1.454 (3) | | N(2)-N(1)-C(6) | 120.6 (2) |
| C(3)-N(4) | 1.360 (3) | | N(2)-C(3)-N(4) | 111.9 (2) |
| S(5)-C(5) | 1.689(2) | | N(1)-C(5)-N(4) | 103.6(2) |
| O(7)-C(7) | 1.433 (3) | | N(4)-C(5)-S(5) | 127.8 (2) |
| C(7)-C(8) | 1.539 (3) | | O(7)-C(7)-C(6) | 104.8 (2) |
| C(9)-C(14) | 1.393 (4) | | C(6)-C(7)-C(15) | 113.6 (2) |
| Cl(10)-C(10) | 1.743 (3) | | C(6)-C(7)-C(8) | 109.9 (2) |
| C(11)-C(12) | 1.384 (4) | | C(9)-C(8)-C(7) | 117.2 (2) |
| C(13)-C(14) | 1.391 (4) | | C(14)-C(9)-C(8) | 119.6 (2) |
| C(15)-C(16) | 1.490 (4) | | C(11)-C(10)-C(9) | 122.4 (2) |
| C(16)-C(17) | 1.521 (4) | | C(9)-C(10)-Cl(10) | 120.1 (3) |
| N(1)-N(2) | 1.377 (3) | | C(13)-C(12)-C(11) | 119.9 (3) |
| N(2)-C(3) | 1.301 (4) | | C(13)-C(14)-C(9) | 121.9 (3) |
| N(4)-C(5) | 1.361 (3) | | C(16)-C(15)-C(7) | 123.2 (2) |
| C(6)-C(7) | 1.533 (3) | | C(16)-C(15)-Cl(15) | 115.7 (2) |
| C(7)-C(15) | 1.536 (3) | | C(7)-C(15)-Cl(15) | 112.2 (2) |
| C(8)-C(9) | 1.515 (3) | | C(15)-C(17)-C(16) | 59.0(2) |
| C(9)-C(10) | 1.395 (4) | | C(5)-N(1)-C(6) | 126.6 (2) |
| C(10)-C(11) | 1.382 (4) | | C(3)-N(2)-N(1) | 103.5 (2) |
| C(12)-C(13) | 1.379 (5) | | C(3)-N(4)-C(5) | 108.2 (2) |
| Cl(15)-C(15) | 1.773 (3) | | N(1)-C(5)-S(5) | 128.5 (2) |
| C(15)-C(17) | 1.503 (4) | | N(1)-C(6)-C(7) | 113.3 (2) |
| | | | O(7)-C(7)-C(15) | 108.9 (2) |
| | | | O(7)-C(7)-C(8) | 111.7 (2) |
| | | | C(15)-C(7)-C(8) | 108.1 (2) |
| | | | C(14)-C(9)-C(10) | 116.5 (2) |
| | | | C(10)-C(9)-C(8) | 123.9 (2) |
| | | | C(11)-C(10)-Cl(10) | 117.4 (2) |
| | | | C(10)-C(11)-C(12) | 119.5 (3) |
| | | | C(12)-C(13)-C(14) | 119.8 (3) |
| | | | C(16)-C(15)-C(17) | 61.1 (2) |
| | | | C(17)-C(15)-C(7) | 120.6 (2) |
| | | | C(17)-C(15)-Cl(15) | 115.1 (2) |
| | | | C(15)-C(16)-C(17) | 59.9 (2) |
c) a unit cell having the following dimensions
| | |
|---|---|
| a = 9.8927(8) Å | α = 90° |
| b = 9.5635 (8) Å | β = 92.651 (6)° |
| c = 16.4448 (10) Å | γ = 90° |
d) a melting point of 138.3°C
and
e) a particle density of 1.471 Mg/m³.

2. Process for preparing the crystal form II of the triazole derivative of the formula (A) according to Claim 1, **characterized in that** the crystal form I of this substance is treated in the presence of
• water and/or
• one or more aliphatic alcohols having 1 to 10 carbon atoms and/or
• one or more dialkyl ketones having 1 to 4 carbon atoms in each alkyl moiety and/or
• one or more alkyl alkylcarboxylates having 1 to 4 carbon atoms in each alkyl moiety
at temperatures between 0°C and 90°C.

3. Microbicidal compositions, **characterized in that** they comprise a triazole derivative of the formula (A) according to Claim 1 in the crystal form II, in addition to extenders and/or surfactants.

4. Use of crystal form II of the triazole derivative of the formula (A) according to Claim 1 for controlling unwanted microorganisms.

5. Method for controlling unwanted microorganisms, **characterized in that** crystal form II of the triazole derivative of the formula (A) according to Claim 1 is applied to the microorganisms and/or their habitat.

6. Process for preparing microbicidal compositions, **characterized in that** crystal form II of the triazole derivative of the formula (A) according to Claim 1 is mixed with extenders and/or surfactants.

## Revendications

1. Modification cristalline II du 2-[2-(1-chlorocyclopropyl)-3-(2-chlorophényl)-2-hydroxypropyl]-2,4-dihydro-3H-1,2,4-triazol-3-thion de formule 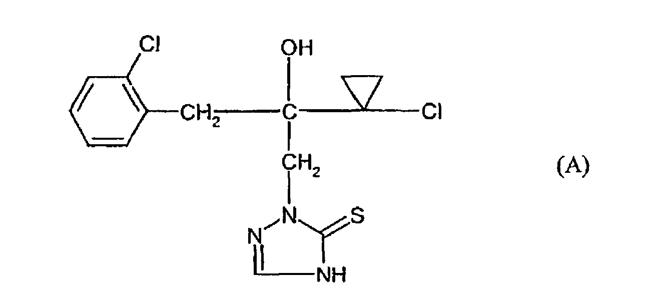 **caractérisé par**
a) des pics maximaux dans le spectre Raman aux nombres d'ondes suivants [en cm⁻¹]
| | | | |
|---|---|---|---|
| 3220 | 1375 | 1101 | 876 |
| 3151 | 1351 | 1065 | 869 |
| 3063 | 1339 | 1052 | 849 |
| 3016 | 1324 | 1038 | 822 |
| 2927 | 1290 | 1032 | 796 |
| 1542 | 1220 | 1001 | 782 |
| 1476 | 1204 | 963 | 759 |
| 1455 | 1184 | 954 | 752 |
| 1445 | 1169 | 922 | 748 |
| 1424 | 1137 | 912 | 725 |
| 1407 | 1123 | 889 | 680 |
b) les longueurs de liaison [en Å] et les angles de liaison [en °] suivants
| **Liaison** | **Longueur [Å]** | | **Liaisons** | **Angle [°]** |
|---|---|---|---|---|
| N(1)-C(5) | 1,350 (3) | | C(5)-N(1)-N(2) | 112,8 (2) |
| N(1)-C(6) | 1,454 (3) | | N(2)-N(1)-C(6) | 120,6 (2) |
| C(3)-N(4) | 1,360 (3) | | N(2)-C(3)-N(4) | 111,9 (2) |
| S(5)-C(5) | 1,689 (2) | | N(1)-C(5)-N(4) | 103,6 (2) |
| O(7)-C(7) | 1,433 (3) | | N(4)-C(5)-S(5) | 127,8 (2) |
| C(7)-C(8) | 1,539 (3) | | O(7)-C(7)-C(6) | 104,8 (2) |
| C(9)-C(14) | 1,393 (4) | | C(6)-C(7)-C(15) | 113,6 (2) |
| Cl(10)-C(10) | 1,743 (3) | | C(6)-C(7)-C(8) | 109,9 (2) |
| C(11)-C(12) | 1,384 (4) | | C(9)-C(8)-C(7) | 117,2 (2) |
| C(13)-C(14) | 1,391 (4) | | C(14)-C(9)-C(8) | 119,6 (2) |
| C(15)-C(16) | 1,490 (4) | | C(11)-C(10)-C(9) | 122,4 (2) |
| C(16)-C(17) | 1,521 (4) | | C(9)-C(10)-Cl(10) | 120,1 (3) |
| N(1)-N(2) | 1,377(3) | | C(13)-C(12)-C(11) | 119,9 (3) |
| N(2)-C(3) | 1,301 (4) | | C(13)-C(14)-C(9) | 121,9 (3) |
| N(4)-C(5) | 1,361 (3) | | C(16)-C(15)-C(7) | 123,2 (2) |
| C(6)-C(7) | 1,533 (3) | | C(16)-C(15)-Cl(15) | 115,7 (2) |
| C(7)-C(15) | 1,536 (3) | | C(7)-C(15)-Cl(15) | 112,2 (2) |
| C(8)-C(9) | 1,515 (3) | | C(15)-C(17)-C(16) | 59,0 (2) |
| C(9)-C(10) | 1,395 (4) | | C(5)-N(1)-C(6) | 126,6 (2) |
| C(10)-C(11) | 1,382 (4) | | C(3)-N(2)-N(1) | 103,5 (2) |
| C(12)-C(13) | 1,379 (5) | | C(3)-N(4)-C(5) | 108,2 (2) |
| Cl(15)-C(15) | 1,773 (3) | | N(1)-C(5)-S(5) | 128,5 (2) |
| C(15)-C(17) | 1,503 (4) | | N(1)-C(6)-C(7) | 113,3 (2) |
| | | | O(7)-C(7)-C(15) | 108,9 (2) |
| | | | O(7)-C(7)-C(8) | 111,7 (2) |
| | | | C(15)-C(7)-C(8) | 108,1 (2) |
| | | | C(14)-C(9)-C(10) | 116,5 (2) |
| | | | C(10)-C(9)-C(8) | 123,9 (2) |
| | | | C(11)C(10)-Cl(10) | 117,4 (2) |
| | | | C(10)-C(11)-C(12) | 119,5 (3) |
| | | | C(12)-C(13)-C(14) | 119,8 (3) |
| | | | C(16)-C(15)-C(17) | 61,1 (2) |
| | | | C(17)-C(15)-C(7) | 120,6 (2) |
| | | | C(17)-C(15)-Cl15) | 115,1 (2) |
| | | | C(15)-C(16)-C(17) | 59,9 (2) |
c) une cellule cristalline de base présentant les dimensions suivantes
| | |
|---|---|
| a = 9,8927 (8) Å | α = 90 ° |
| b = 9,5635 (8) Å | β = 92,651 (6) ° |
| c = 16,4448 (10) Å | γ = 90 ° |
d) un point de fusion de 138,3 °C
et
e) une densité absolue de 1,471 Mg/m³.

2. Procédé pour fabriquer la modification cristalline II du dérivé triazol de formule (A) selon la revendication 1, **caractérisé en ce que** l'on traite la modification cristalline I de cette substance en présence
• d'eau et/ou
• d'un ou plusieurs alcools aliphatiques avec 1 à 10 atomes de carbone et/ou
• un ou plusieurs dialkylcétones avec 1 à 4 atomes de carbone dans chaque groupe alkyle et/ou
• un ou plusieurs esters d'alkyle d'acide alkylcarbonique avec 1 à 4 atomes de carbone dans chaque groupe alkyle
à des températures comprises entre 0 °C et 90 °C.

3. Agent microbicide **caractérisé par** une teneur en dérivé triazol de formule (A) selon la revendication 1 dans la modification cristalline II avec des agents de charge et/ou des agents tensioactifs.

4. Utilisation de la modification cristalline II du dérivé triazol de formule (A) selon la revendication 1 pour lutter contre les micro-organismes indésirables.

5. Procédé pour lutter contre les micro-organismes indésirables, **caractérisé en ce qu'**on applique la modification cristalline II du dérivé triazol de formule (A) selon la revendication 1 sur les micro-organismes et/ou leur biotope.

6. Procédé de fabrication d'agents microbicides, **caractérisé en ce que** l'on mélange la modification cristalline II du dérivé triazol de formule (A) selon la revendication 1 avec des agents de charge et/ou des agents tensioactifs.
